# EUROPEAN PATENT APPLICATION

(11) **EP 1 293 200 A1**
(43) Date of publication of application: **19.03.2003**
(21) Application number: 01904342.1
(22) Date of filing: 08.02.2001
(51) Int. Cl.: A61K 31/122, A61P 25/00, A61P 25/28

(54) **NERVE GROWTH FACTOR ACTIVITY POTENTIATING AGENTS**

(30) Priority: 08.02.2000 JP 2000030546
(71) Applicant: HISAMITSU PHARMACEUTICAL CO. INC., Tosu-shi, Saga-ken 841-0017 (JP)
(72) Inventor: GOTO, Takeshi Hisamitsu Pharmaceutical Co., Inc., Tsukuba-shi Ibaraki 305-0856 (JP); ITOYAMA, Toshio Hisamitsu Pharmaceutical Co., Inc., Tsukuba-shi Ibaraki 305-0856 (JP); FUKUSHIMA, Hidenao Hisamitsu Pharmaceu. Co., Inc., Tsukuba-shi Ibaraki 305-0856 (JP); KAMEI, Yuto, Karatsu-shi, Saga 847-0863 (JP)
(74) Representative: Flaccus, Rolf-Dieter, Dr.
(86) International application number: JP0100878
(87) International publication number: WO01058437

(57) **Abstract**

Agents potentiating nerve growth factor activity for treating Alzheimer's disease which contain as the active ingredient dodecatrienoic acid derivatives represented by the general formula (1) wherein X represents hydrogen, CHO, COOH, COOR (wherein R represents an ester residue or an addition salt comprising an alkali metal salt or an organic acid salt group), provided that in case where X is COOH, it is bonded to the carbon atom at the position marked with * so as to form a lactam O=C-O-.

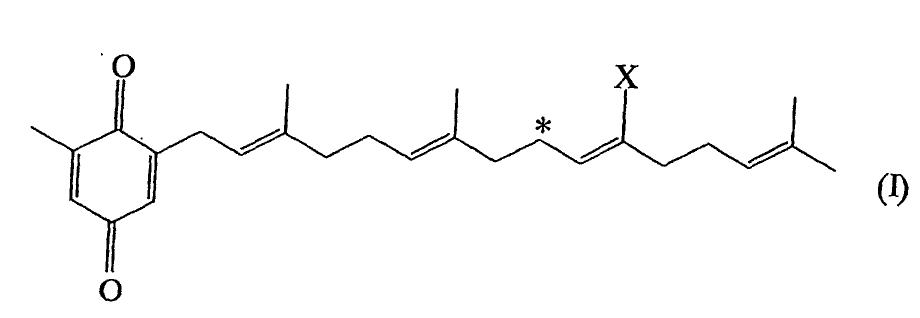

## Description

### [Technical Field of the Invention]

The invention relates to a nerve growth factor activity potentiator

### [Background Art]

The hypofunction in the brain is recognized from two points of view : reduction in numbers of nerve cells and disappearance of their network. The network of nerve cells is formed, in which dendrites elongating from a nerve cell combine with dendrites, axial filaments or cell bodies in many nerve cells and synapses are constituted, making various information processing including memory, learning and perception possible. Usually, although several ten thousand synapses per one nerve cell exist, a remarkable reduction of dendrites occurs in a nerve cell which is aged or damaged. As a result, the number of synapses decreases and thus lowering of a brain function is brought about. Then, cells aged or damaged, in which the atrophy and degeneration of dendrites or axial filaments progress slowly, finally die. Although reduction of nerve cells and change of a synapse network progresses steadily, accompanying aging or the progress of disease, this change does not always bring about lowering of the function. For example, it is known that even if a part of the nerve cell network is broken, nerve cells remaining in the neighborhood elongate dendrites whereby a new synapse is formed, having function to compensate a lowered function of the nerve circuit network to some degree.

Currently, there is a tendency that the increase of elderly population becomes increasingly serious in developed countries, and it is anticipated that in our country the proportion of elderly aged people among the total population becomes the No.1 of the world. As for the increase of senile dementia accompanying such aging, because especially Alzheimer type dementia is an intractable disease, which is progressively lethal, with a pathological characteristics such as degeneration-dropout or senile plaque, up to now research into Alzheimer type dementia has been carried out extensively, making a lot of facts become clear. For example, facts such as the appearance of abnormal structural bodies including senile plaque (Masters et al., Proc. Natl. Acad. Sci. USA, 82, 4245-4249, 1985; Seuber et al., Nature, 359, 325-327,1992), Alzheimer's fibrillary (Kondo et al., Neuron, 1, 827-834, 1988) or the like, accumulation of aluminum in the brain (Kawahara et al., Koushu Eisei Kenkyu, 42, 520-525, 1993) and deficit of neurotrophic factor (Hefti et al., Ann. Neurol., 13, 109-110, 1983). However, up to now a clear elucidation of the pathogenesis or a therapeutic method have not been established yet, making its establishment an urgent problem.

The neurotrophic factor is a physiologically active substance with a diffusing property, which is necessary for differentiation and survival sustainment for nerve cells (Thoenen & Edgar, Science, 229, 238-242, 1985), and almost all of them are glycoprotein. It is considered that each neurotrophic factor is synthesized in a projecting site of each nerve cell's axial filament, that is, glia cell or a vicinal cell, and is taken in the cell body from the end of the axial filament by antiport, showing various pharmacological actions. The neurotrophic factor found first is Nerve Growth Factor (NGF, Thoenen & Brade, Physiol. Rev., 60, 1284-1335, 1980) acting to sympathetic nerves, sensory nerves at a fetal early period, or cholinergic nerve cells in the frontal basal nuclei. After the finding of NGF reseach of a neurotrophic factor was extensively carried out, and Brain Derived Neurotrophic Factor acting to sensory nerve cells insensitive toward NGF (BDNF; Brade et al., EMBO. J. 1, 549-553, 1982), Cilialy Neurotrophic Factor (CNTF; Watters & Hendy, J. Neurochem., 49, 705-713, 1987), further, Neurotrophin-3 belonging to these gene families (NT-3; Hohn et al., Nature, 344, 339-341, 1990; Maisonpierre et al., Science, 247, 1446-1451,1990), Neurotrophin-4 (NF-4) and Neurotrophin-5 (NF-5) (Davies et al., J. Neurosci., 13, 4961-4967, 1993), etc., were newly found.

Further, it has been made evident that Fibroblast Growth Factor (FGF; Morrison et al., Proc. Natl. Acad. Sci. USA, 83, 7537-7541,1986), Epidermal Growth Factor (EGF; Morrison et al., Science, 238, 72-75, 1987) and Insulin-Like Growth Factor (Aizerman & Vellis, Brain. Res. 406, 32-42, 1987), which are the known cell growth factors, which also keep the survival of nerve cells under culturing, making a neurotrophic factor like action. Additionally, except these glycoproteins it is reported that thyroxine of thyroid hormone which is the amino acid containing iodine (Hayashi & Payel, Dev. Brain. Res., 36, 109-120, 1987) and estradiol which is a steroid hormone (Arimatsu & Hatanaka, Dev. Brain. Res., 26, 151-159, 1986) also show a neurotrophic factor like action. Thus, it is an unquestionable fact that various neurotrophic factors are necessary for keeping the function or survival of nerve cells.

According to the neurotrophic factor deficiency theory of Hefti et al., (Hefti et al., Annn. Neurol., 13, 109-110, 1983) it is considered that the degeneration-atrophy of nerve cells due to the reduction of a neurotrophic factor synthesis concerned with the survival sustainment and the differentiation acceleration of nerve cells, or the dropout of nerve cells due to the apoptosis or necrosis of nerve cells occurs, producing the onset of Alzheimer type dementia. In these circumstances, it was proved that NGF, one of neurotrophic factors, acts to large cholinergic nerve cells of cerebral Meynert nuclei, showing the survival sustainment and differentiation acceleration action of nerve cells (Whitehouse et al., Science, 215, 1237-1239, 1982; Bartus et al., Science, 217, 408-417,1982; Fibiger et al., TINS, 14, 220-223,1991), and it was suggested that there was a close relationship between NGF action and Alzheimer dementia (Brade, Neuron, 2, 1525-1534, 1989). Actually, when NGF was administered directly into the brain of Alzheimer dementia patient, the disease condition was recovered transiently (Olson et al., J. Neural. Transm., 4, 79-95, 1992). However, Since NGF is a glycoprotein of the molecular weight 140,000, it can not pass through blood-brain barrier, and there are problems such as its absorption efficiency or stability in the blood or digestive tract. Therefore, it is anticipated that use of NGF itself as a pharmaceutical preparation is extremely difficult.

Currently, the development of drugs to treat the disease has been tried, and among them an acetyl choline esterase inhibitor is being used as an anti-dementia agent, since the reduction of acetyl choline content in cerebral cortex projection nerve making Meynert nuclei as nucleus originis is observed at the onset. However, although it is possible to some degree to delay the progress of Alzheimer type dementia due to the fact the reduction of brain function accompanied with nerve cell death is irreversible, it is extremely difficult to improve the disease condition and it is whatever only a symptomatic treatment. Further, since acetyl choline is a neurotransmitter in the parasympathetic nerve system, concerned are not only side effects related to circulatory organs such as syncope, bradycardia, cardiac block or myocardial infarction but severe side effects such as peptic ulcer, hepatopathy, hepatitis or cerebral attack. On this background, it has been longed to provide a low molecular compound, which increases the NGF activity, has a molecular size possible for pass through the blood-brain barrier and is reduced in its side effect.

Further, except the above Alzheimer dementia, illustrative of diseases accompanying dementia are such as juvenile Alzheimer dementia, Pick disease, Huntington chorea, Parkinson disease, spinocerebelar degeneration, progressive supranuclear palsy and intractable epilepsy which are classified as nerve degenerative diseases, multiple cerebral infarction, Binswanger type subcortical encephalopathy and chronic subdural hematoma which are classified as angiopathic diseases, multiple sclerosis and collagen disease which are classified as infectious diseases, primary brain tumor, metastatic brain tumor and limbic encephalitis which are classified as brain tumors, and manic-depressive psychosis, schizoid and reactive psychosis which are classified as mental diseases.

Meanwhile, in case a candidate for a pharmaceutical preparation is explored, products of animals and plants, which live in land and water, are noted, and a method to explore these has currently been known. Meanwhile, not withstanding that the ocean occupies 70% of the earth surface and about 80% of living species live in the ocean, so far the development objects for pharmaceutical preparations by the previous method have been confined almost to animals and plants on land.

Up to now, as to substances produced by *Sargassum macrocarpum,* brown alga, several types have been purified, and chemical structures of dodecatrienoic acid derivatives used in the invention have already been reported (Kusumi et al., Chem. Lett., 3, 277-278, 1979; Segawa et al., Chem. Lett., 7, 1365-1366, 1987). However, the pharmacological activities of the extraction ingredients including the 2,6,10-dodecatrienoic acids used in the invention have not been reported yet.

### [Disclosure of the Invention]

The object of the invention is to provide a nerve growth factor activity potentiator consisting of a low molecular compound in which current problems are solved and its side effect is low. The nerve growth factor activity potentiating agent mentioned here includes one having a neurite elongation, brain function activation, memory improvement or anti-dementia actions, or the like in a broader sense, and preparations containing these obviously mean a neurite elongation, brain function activation, memory improvement or anti-dementia agents.

The inventors made extensive researches to solve the above problems and focused on marine creatures, especially seagrasses living in the seas near Japan from the view point that marine organisms whose living environment is extremely different from that of land organisms have in a high possibility body ingredients different from substances derived from terrestrial organisms, reflecting difference of metabolism in the body and have a great potential for a development of a novel pharmaceutical preparation. Thus, a result of a wide range extraction and a random screening test, the inventors found out for the first time that the 2,6,10-dodecatrienoic acid produced by *Sargassum macrocarpum*, brown alga, or salts thereof are the substances potentiating the activity of Nerve Growth Factor (NGF), and accomplished the invention.

Namely, the invention relates to nerve growth factor activity potentiators which contain as an active ingredient dodecatrienoic acid derivatives represented by the general formula (I); 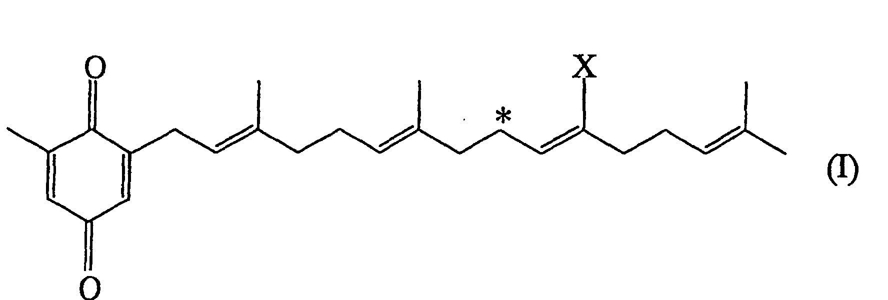 wherein X represents hydrogen, CHO, COON, COOR (wherein R represents an ester residue or an addition salt comprising an alkali metal salt or an organic acid salt group), provided that in case X is COOH, it is bonded to the carbon atom at the position marked with * so that it forms a lactam O=C-O-.

The invention also relates to nerve growth factor activity potentiators comprising an extraction ingredient extracted from *Sargassum macrocarpum* or *Jania adharens.*

Further, the invention also relates to agents potentiating nerve growth factor activity patentiators wherein the extraction ingredient contains 2,6,10-dodecatrienoic acid or salts thereof.

The invention also relates to a pharmaceutical composition for a dementia disease containing the above nerve growth factor activity potentiators.

In the formula (I) X means a hydrogen atom, a formyl group which is CHO, a carboxyl group which is COOH or basic salts thereof, an ester residue represented by COOR wherein R is an alkyl, a substituted or unsubstituted benzyl, a substituted phenyl or unsubstituted phenyl groups, or the like, or an addition salt comprising an alkali metal salt or an organic acid salt wherein R is sodium, potassium, calcium, ammonium or the like.

As a preparation method of dodecatrienoic acid derivatives or salts thereof having a potentiation on nerve growth factor activity, the following method can be illustrated, though it is not limited thereto. That is, although 2,6,10-dodecatrienoic acid or salts thereof, which are dodecatrienoic acid derivatives, can be obtained by extraction, purification and isolation from *Sargassum macrocarpum*, brown alga, in the meantime the preparation method of these compounds and their derivatives has been disclosed in Chem. Lett., 3, 277-278, 1979 as well as Chem. Lett., 7, 1365-1366, 1987, and methods such as synthesis in accordance with this method can be used. Further, making these compounds a seed compound, a more effective derivative (lead compound) can be created by making the characteristics on the chemical structures clear with the examination of structure-activity relationship, etc., as well as by making full use of synthetic methodologies such as combinatorial chemistry, etc.

In case dodecatrienoic acid derivatives used in the invention is used in a therapy, they are provided as a pharmaceutical composition by mixing appropriately excipients or the like which are pharmaceutically accepted in general.

In the pharmaceutical composition one or more of pharmaceutically acceptable carriers are mixed to make a known preparation form, illustrative of carriers being, for example, fillers, extenders, binders, disintegrators, surface active agents, lubricants and moisturizing agents. The representative examples of forms of a pharmaceutical composition include tablets, granules, powders, pills, suppositories, capsules, syrups, emulsions, suspensions, liquids, injections, pastes, ointments, creams, gels, gelatinized creams, lotions, fomentations, plasters, liniments, aerosols, buccals, eye drops, nasal drops, etc., and can stably be administered systemically or locally as well as orally or parenterally.

In case oral preparation such as tablets and granules are formulated, as excipients carbohydrates such as lactose, sugar, glucose, mannitol and sorbitol, starches such as corn starch, potato starch and dextrin, micro-crystalline cellulose, gum arabic, dextrin, pullulan, light anhydrous silisic acids, aluminum silicate, magnesium metasilycate aluminate, magnesium silicate, calcium phosphate, calcium carbonate, calcium sulfate, etc., as disintegrators carboxymethyl cellulose, sodium carboxymethyl cellulose, calcium carboxymethyl cellulose, hydroxypropyl cellulose, crystalline cellulose, ethyl cellulose, sodium carboxymethyl starch, sodium croscarmellose, etc., as binders polyvinyl pyrrolidone, polyvinyl alcohol, hydroxypropyl cellulose, etc., as lubricants talc, stearic acid, magnesium stearate, calcium stearate, etc., and as the others polyethylene glycols, propylene glycols and dyes can be mixed appropriately. Further, if desired, prepared are solid preparations applied by a usual coating such as a sugar coated solid preparation, a gelatin encapsulated enteric solid preparation, a film coated solid preparation, a double-layer solid preparation or a multilayer solid preparation.

In case pills are formulated, they can be prepared by a known method using a known carrier. As such a carrier, an excipient such as glucose, lactose, starch, cocoa butter, hardened vegetable oil, kaolin, talc or the like, and a disintegrator such as laminaran agar, and the like can appropriately be mixed.

In case suppositories are formulated, they can be prepared by a known method using a known suppository base. Such a suppository base includes a lipophilic base, an aqueous base, an emulsified base or the like, and they can be used by an appropriate selection. Examples of such a suppository base include cocoa butter, hydrogenated peanut oil, hydrogenated coconut oil, polyethylene glycol, Monolen, Tulen, Prulonic or the like. Further, additives such as a local anesthetic, antihistamine, local astringent, sulfa drug, antibiotic, wound healing drug, surface active agent, vitamin, crude drug extract, bile acid, antiseptic, excipient, sorbefacient, amino acid or the like can appropriately be mixed.

As a base used for hard capsules or soft capsules in a capsule formulation, a plastisizer such as gelatin, glycerin, sorbitol, propylene glycol, sucrose, gum arabic or the like, a colorant such as dye, titanium oxide or the like, a preservative such as a methyl, ethyl or propyl p-hydroxybenzoates (parabens) or the like, an aromatic substance and other excipient,etc., are appropriately formulated, whereby they can be prepared.

In case injections are formulated, they can be prepared by a known method using a known dilution agent and the like. As for an injection a septic liquid, emulsions and suspensions are used, whereby it is preferable to make them isotonize with the blood. Illustrative of a dilution agent used to make them in forms of liquids, emulsions and suspensions are, for example, water, an aqueous lactic acid solution, ethyl alcohol, propylene glycol, polyoxyethyl sorbitan fatty acid ester, etc., and a sufficiant amount of sodium chloride, glucose or glycerin to prepare an isotonic solution, a solubilizing agent, a buffer substance, a soothing agent, additionally a colorant, a preservative, a flavoring, a sweetner,etc., are appropriately formulated, whereby it can be prepared.

As for formulations of syrups, emulsions, suspensions and liquids, a non-ionic active agents such as water, ethanol, glycerin, sorbitol, polyethlene glycol, propylene glycol, glycerol monostearate, polyoxy stearate, lauromacrogol, sorbitan oleate, polysorbate 80 or sucrose fatty acid ester, an anionic active agents such as stearyl triethanolamine or sodium lauryl sulfate, and a cationic active agents such as benzalkonium chloride or benzethonium chloride as a solubilizing agent or an emulsifying agent, or a surface active agent such as the above non-ionic active agent, anionic agent or cationic agent, a polyvinyl type compound such as polyvinyl alcohol or polyvinyl pyrrolidone, a cellulose derivative such as sodium carboxymethyl cellulose, methyl cellulose, hydroxymethyl cellulose, hydroxyethyl cellulose or hydroxypropylmethyl cellulose, additionally gum arabic, gelatin or the like as a dispersing agent, aluminum magnesium silicate, colloidal aluminum magnesium silicate hydrate, bentonite, kaolin or microcrystalline cellulose as a viscous agent, parabens, benzalkonium chloride or benzethonium chloride as a preservative, furctose, invert sugar, cocoa, citric acid, ascorbic acid, fruit juice as a flavoring or a sweetner, and the other excipients, etc., are appropriately formulated, whereby they can be prepared.

The above liquids can be made to an aerosol composition by mixing a usual propellant agent. As the propellant agent, illustrative are dimethyl ether, a liquefied petroleum gas, nitrogen gas, nitrous oxide gas, carbon dioxide gas, CFCs substitute, etc., which are conventionally used for aerosols. A compressed air can also be used without using a propellant agent. Further, a mixed gas of these can be used. If desired, an aromatic substance or an ingredient conventionally used in a drug for external use, for example, such as 1-menthol, tocopherol acetate, furthermore, oil including a plant oil, an animal oil or the like such as castor oil or squalene are appropriately formulated, whereby they can be prepared.

Also, in case ointments are formulated, they can be prepared by a known method using a known ointment base. As the ointment base, illustrative are higher fatty acids or esters thereof such as adipic acid, myristic acid, palmitic acid, stearic acid, oleic acid, adipate, palmitate, diethyl sebacate, hexyl laurate and cetyl isooctanoate, waxes such as whale wax, beeswax and Sercin, surface active agents such as polyoxyethylene alkylether phosphate, higher alcohols such as cetanol, stearyl alcohol and cetostearyl alcohol, silicon oils such as dimethyl polysiloxane, methylphenyl polysiloxane, glycolmethyl polysiloxane and silicon-glycol copolymer, hydrocarbons such as propylene carbonate, diisopropyl adipate, crotamiton, Azone and pyrothiodecane, moisturizing agents such as glycerin, propylene glycol, butylene glycol and sorbitol, anti-eruption agents and other additives, and the preparation can be made by an appropriate formulation therefrom.

In case creams are formulated, they can be prepared by a known method using a known cream base. Examples include as the cream base higher fatty acid esters such as myristate, palmitate, diethyl sebacate, hexyl laurate and cetyl isooctanoate, lower alcohols such as ethanol and isopropanol, hydrocarbons such as liquid paraffin and squalene, polyalcohols such as propylene glycol and 1,3-butylene glycol, higher alcohols such as 2-hexyldecanol, cetanol and 2-octyl decanol, as emulsifying agents polyoxyethylene alkyl ethers, fatty acid esters, polyethylene glycol fatty acid esters, etc., as antiseptics p-hydroxy benzoic acid esters, as sorbefacients propylene carbonate, diethyl cebacate, diisopropyl adipate, crotamiton, Azone and pyrothiodecane, anti-eruption agents and other additives, and the preparation can be made by an appropriate formulation from these.

In case gels are formulated, they can be prepared by a known method using a known gel base. As the gel base, illustrative are lower alcohols such as ethanol and isopropyl alcohol, gelatinizing agents such as water, carboxyvinyl polymer, hydroxyethyl cellulose, hydroxypropyl cellulose, methyl cellulose, ethyl cellulose, carboxymethyl cellulose and propyleneglycol alginate, neutralizing agents such as triethanolamine, diisopropanolamine and sodium hydroxide, surface active agents such as sorbitan sesquioleate, sorbitan trioleate, sorbitan monooleate, sorbitan monostearate, sorbitan monolaurate, polyethlene glycol monostearate, polyoxyethylene nonylphenyl ether, polyoxyethylene cetyl ether and polyoxyethylene lauryl ether, sorbefacients such as propylene carbonate, diethyl cebacate, diisopropyl adipate, crotamiton, Azone, propylene glycol and pyrothiodecane, anti-eruption agents and the other additives, and the preparation can be made by an appropriate formulation from these.

In case gelatinized creams are formulated, the above creams were added with a gelatinizing agent such as carboxyvinyl polymer, hydroxyethyl cellulose, hydroxypropyl cellulose, methy cellulose, ethyl cellulose or carboxymethyl cellulose, adjusting the pH to 4-9, preferably 5-7 by adding a neutralizing agent such as diisopropanolamine, triethanolamine or sodium hydroxide to give the preparation.

In case fomentations are formulated, they can be prepared by a known method using a known fomentation base. Illustrative as thickeners are aqueous synthetic polymers such as sodium polyacrylate, polyacryic acid, Poval, polyvinyl pyrrolidone, polyethylene oxide and polyvinyl methacrylate, natural products such as gum arabic, starch and gelatin, methyl cellulose, hydroxypropyl cellulose, alginic acid, sodium alginate, sodium carboxymethyl cellulose, etc., and further illustrative are wetting agents such as urea, glycerin, propylene glycol, butylene glycol and sorbitol, fillers such as kaolin, zinc oxide, talc, -titanium, bentonite, epoxy resins, organic acids (fillers such as tartaric acid, maleic acid, maleic anhydride and succinic acid), calcium, magnesium and aluminum, solubilizin agents such as water, propylene carbonate, crotamiton and diisopropyl adipate, tackifying agents such as rosin, ester gum, polybutene and polyacrylate, additionally anti-eruption agents such as diphenhydramine hydrochloride, chlorphenyramine maleate, glycyrrhizic acid, dexamethasone, betamethasone, fluocinolone acetonide, and additives such as salicylic acid, methy salicylate, glycol salicylate, 1-menthol, camphor, 4-hydroxy-3-methoxybenzyl nonylic acid amide, thymol, pepper extract, mint oil, Azone and pyrothiodecane, and the preparation can be made by an appropriate formulation from these.

In case plasters are formulated, they can be prepared by a known method using a known plaster base. Illustrative as the plaster base are polymer bases such as acrylic type compositions which are copolymers of methacrylate, acrylonitrile, and vinyl monomers such as vinyl acetate or vinyl propionate, silicone resin, polyisobutylene rubber, polyisoprene rubber, natural rubber, acrylic rubber, styrene-butadiene-styrene block copolymer and styrene-isoprene-styrene block copolymer, oils or higher fatty acids such as almond oil, olive oil, camellia oil, bersic oil, olein oil, liquid paraffin and polybutene, adhesive agents such as rosin, rosin-denaturated maleic acid and hydrogenated rosin ester, anti-eruption agents, and additives such as dl-camphor, 1-menthol, thymol, 4-hydroxy-3-methoxybenzyl nonylic acid amide, pepper tincture, mint oil, crotamiton, peppermint oil, Azone and pyrothiodecane, and the preparation can be made by an appropriate formulation from these.

In case liniments are formulated, they can be prepared by a known method using a known liniment base. Illustrative as the liniment base are alcohols such as ethanol, propanol, isopropanol, polyethylene glycol, propylene glycol and butylene glycol, fatty acid esters such as each ester of adipic acid, sebacic acid or myristic acid, surface active agents such as polyoxyethylene alkyl ether, neutralizing agents, viscosity donating agents such as methyl cellulose, carboxyvinyl polymer and hydroxypropyl cellulose, anti-eruption agents, and additives such as salicylic acid, methyl salicylate, glycol salicylate, 1-menthol, camphor, mint oil, pepper extract, 4-hydroxy-3-methoxybenzyl nonylic acid amide, thymol, crotamiton, Azone, propylene carbonate, diisopropyl adipate and pyrothiodecane, and the preparation can be made by an appropriate formulation from these.

In case the decatrienoic acid derivatives used in the invention are used as a pharmaceutical composition, the doses depend on the sex, body weight, age and specific disease condition of a patient, usually they being administered in doses between approximately 0.1 and 1000mg/kg in one or more times per day.

As for the decatrienoic acid derivatives used in the invention, especially, a pharmaceutical composition making the decatrienoic acid derivatives shown by the above formula (I) an active ingredient can enhance the NGF activity, and therefore they can be used as a nerve growth factor activity potentiator.

### [Brief Description of Drawings]

### [Fig. 1]

This figure describes the neurite elongation activity potentiation in PC12D cells when the methanol extraction ingredient of *Sargassum macrocarpum* was added under NGF deficiency (10ng/ml). The arrows in the figure indicate the doses in which the cell toxicity was observed due to the addition of *Sargassum macrocarpum* methanol extraction ingredient.

### [Fig. 2]

A: This figure describes the neurite elongation activity potentiation in Neuro-2a cells when the methanol extraction ingredient of *Sargassum macrocarpum* was added under NGF deficiency (10ng/ml). The arrows in the figure indicate the doses in which the cell toxicity was observed due to the addition of *Sargassum macrocarpum* methanol extraction ingredient.
B: This figure describes the neurite elongation activity potentiation in Neuro-2a cells when the methanol extraction ingredient of *Sargassum macrocarpum* was added in the absence of NGF. The arrows in the figure indicate the doses in which the cell toxicity was observed due to the addition of *Sargassum macrocarpum* methanol extraction ingredient.

### [Mode for carrying out the Invention]

In the following, the invention will be explained by the examples, but the invention is not limited to a specific detail of these examples.

Marine algae in Japan coast were collected. In order to search useful materials as a nerve growth factor activity potentiator from their extracts, a random screening for the neurite elongation activity potentiation of 598 samples in 299 species collected was carried out using PC12D cells derived from rat adrenal pheochromocytoma under NGF deficiency.

### 1. Collection of marine algae

Marine algae were collected at a total of 74 places in Hokkaido, Iwate prefecture, Niigata prefecture, Fukuoka prefecture, saga prefecture, Nagasaki prefecture, Kagoshima prefecture and Okinawa prefecture for the period from April 1994 to September 1995. In order to collect samples efficiently, the ebb tide period of spring tide when the height of sea surface becomes lowest was taken. Marine algae on the shore were directly collected from the land, and marine algae growing approximately at less than 5m of water depth were collected at random by a skin diving. In determining these collection places, information on species and amounts of marine algae growing at the site was gathered beforehand, and the geographical distribution was also considered. Marine algae collected at random were divided according to the groups based on difference in forms, and the species were identified, before using them in experiments or subjective to a frozen storage in a deep freezer of -85°C till use in experiments.

### 2. Classification of provided marine algae

According to the result of precise classification the collected marine algae included 95 species of brown algae (in 22 families, 45 genera), 146 species of red algae (in 30 families, 75 genera) and 50 species of green algae (in 10 families, 21 genera), being 299 species in a total.

### 3. Preparation of marine algae extraction liquid

A sample of marine algae was washed with sterilized artificial sea water (ASW, Jamarin Laboratories) 3 times and PBS (phosphate buffer) once, and then 5g of the washed sample in wet weight was taken, added with PBS 20ml, homogenized (8000rpm, 5min, room temperature) by Polytoron (Kinematica), and centrifuged (3000rpm, 20min, room temperature) to give a supernatant as a PBS extraction liquid. The pellet was added with methanol 20ml, homogenized (8000rpm, 5min, room temperature) to give a supernatant as a methanol extraction liquid. The PBS extraction liquid and the methanol extraction liquid were purified by silica gel chromatography, gel permeation chromatography or solvent partition. All the 598 samples of the PBS extraction ingredients and the methanol extraction ingredients of all the 299 species of marine algae provided were diluted to 1/10 referring to the data on the cell toxicity of the marine algae extracts (Harada et al., Biol. Pharm. Bull., 20, 541-546, 1997), and their screening tests were carried out.

### 4. Screening of extraction ingredients by PC12D cells as a measure of a neurite elongation activity

### (1) Experimental method

PC12D cells used were donated by Institute for Developmental Research, Aichi Human Service Center. The culturing of PC12D cells was carried out by plating 5x10³ cells/100µl/well in a 96-well microplate under 95% air and 5% CO₂ at 37°C. As for the medium for cells, E-RDF medium (Kyokuto) containing 10% fetal bovine serum (FBS, Selumex) was used. After culturing for 24 hours the medium was changed to a cell medium added with a low dose of NGF, and then each 5µl of the PBS extraction ingredient and the methanol extraction ingredient of each species of marine alga, which were diluted to 1/10 by the cell medium, was added. After 48 hours from the start of culturing, the effect of each species of alga on the neurite elongation activity was examined by observing under an optical microscope (x200 magnification). As for the measurement of the neurite elongation activity, the number of cells per one visual field and the number of cells having a neurite which was not less than 2 times larger than a cell body was counted, and the neurite elongation activity for the extraction liquid of each species of marine alga was evaluated according to the calculation formula below; neurite elongation activity = number of cells having the neurite per 1 point/total number of cells per 1 point. Two wells per one sample were tested, and the neurite elongation activities were measured for 4 points per well , therefore, the visual fields of 8 points in total was provided, to obtain the mean value. Further, the neurite elongation activities for cells to which medium NGF 2.5ng/ml was added as a negative control and those for cells to which medium NGF 50ng/ml was added as a positive control were measured, for making a comparison was made.

### (2) Results

In the following, the random screening results of the neurite elongation activities in PC12D cells are shown for each 20 samples of the methanol extraction ingredients (Table 1;1-20) and the PBS extraction ingredients (Table 2; 21-40).

**Table 1;**

| Sample No. | Sample name | Neurite elongation activity potentiation |
|---|---|---|
| 1 | *Sargassum horneri* | ± |
| 2 | *Dictyopteris membranacea* | ± |
| 3 | *Patichydictyon coriaceaum* | ± |
| 4 | *Myagropsis myagroides* | ± |
| 5 | *Dictyopeteris undulata* | ± |
| 6 | *Sargassum macrocarpum* | +++ |
| 7 | *Colpomenia siuuosa* | ± |
| 8 | *Colpomenia bullosa* | ± |
| 9 | *Dictyota dichotama* | ± |
| 10 | *Sargassum thunbergii* | ± |
| 11 | *Sargassum yendoi* | + |
| 12 | *Sargassum yezoense* | + |
| 13 | *Zonaria stipitata* | ± |
| 14 | *Agamum cribrosum* | ± |
| 15 | *Eisenia bicyclis* | ± |
| 16 | *Sphaerotrichia divaricata* | ± |
| 17 | *Isige okamurai* | ± |
| 18 | *Sagassum hemipyllum* | ± |
| 19 | *Padina minor* | ± |
| 20 | *Padina arborescens* | ± |

**Table 2;**

| Sample No. | Sample name | Neurite elongation activity potentiation |
|---|---|---|
| 21 | *Ecklomia cave* | ± |
| 22 | *Scytosiphon lomentaria* | ± |
| 23 | *Zonaria diesingiana* | ± |
| 24 | *Sargassum nipponicum* | + |
| 25 | *Hizika fusiformis* | ± |
| 26 | *Sargassum alteronato-pinnatum* | ± |
| 27 | *Sargassum confusum* | ± |
| 28 | *Dictyopteris prolifera* | ± |
| 29 | *Sagassum fulvellum* | ± |
| 30 | *Nemacystis decipiens* | ± |
| 31 | *Sargassum patens* | ± |
| 32 | *Tubinaria ornata* | ± |
| 33 | *Undaria pinnatifida* | ± |
| 34 | *Asparagopsis taxiformis* | ± |
| 35 | *Polysiphonia morrowii* | ± |
| 36 | *Jania adharens* | + + + |
| 37 | *Chondria crassicaulis* | --- |
| 38 | *Hypnea charoides* | *---* |
| 39 | *Amphirosa dilatata* | --- |
| 40 | *Gelidiella acerosa* | --- |

In the Tables, ± means no activity potentiation, + means a weak activity potentiation, +++ means a strong activity potentiation, --- means a strong inhibition.

The strong neurite elongation activity potentiation was confirmed only for two samples of the methanol extraction liquid for *Sargassum macrocarpum*, brown alga and the PBS extraction ingredient for *Jania adharens*, red alga. Their neurite elongation activities showed 0.33 for the methanol extraction ingredient of *Sargassum macrocarpum* and 0.24 for the PBS extraction ingredient for *Jania adharens*, and it was confirmed that they accelerated the neurite elongation activity about 10 times and 8 times respectively compared with the negative control (the neurite elongation activity value for the negative control added with only NGF 2.5ng/ml was about 0.03.). Here, the active ingredient, which was contained in the PBS extraction ingredient for *Jania adharens*, was considered to be the same with that for *Sargassum macrocarpum*. Further, there were few samples in which morphological change, that is, cell toxicity was observed by the addition of the methanol extraction ingredients of marine algae tested. In the meantime, for more cases in their PBS extraction ingredients, the cell toxicity or the inhibition of the neurite elongation rather than the neurite elongation activity potentiation were observed.

### 5. Dose dependency of neurite elongation activity

Subsequently, in order to confirm the excellent neurite elongation activity potentiation of the methanol extraction ingredient for *Sargassum macrocarpum* in which the activity was detected, its dose dependency of the action was examined by use of PC12D cells, respectively varying the concentration of the extraction ingredient to 0, 0.19, 0.38, 0.75, 1.5, 3.0, 6.0, 12.5 (µg/ml) under a low dose of NGF (10ng/ml).

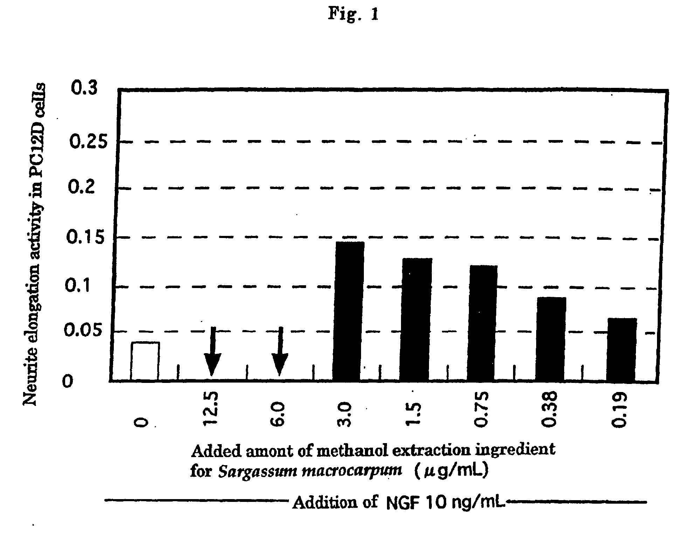

As shown in Fig.1, it turned out that the dose dependent neurite elongation action was confirmed in PC12D cells added in the range of 0.19-3 µg/ml. Namely, the addition of 3.0 µg/ml of the methanol extraction ingredient for *Sargassum macrocarpum* showed the maximum neurite elongation activity potentiation of about 3.6 times compared with the negative control added with only NGF 10 ng/ml, and even in the low dose 0.19 µg/ml a slight potentiation was shown.

Further, the cell body atrophy and furthermore the cell float were observed by the addition of 6 and 12.5 µg/ml of the methanol extraction ingredient for *Sargassum macrocarpum*, confirming the appearance of cell toxicity. Based on the above, it was considered that the methanol extraction ingredient for *Sargassum macrocarpum* accelerated the NGF-TrkA signal cascade by increasing the affinity between NGF and its receptor, TrkA, and elongated the neurites of PC12D cells.

### 6. Confirmation of neurite elongation activity by neuroblastoma Neuro-2a

Further, in order to confirm whether the neurite elongation activity potentiation of the methanol extraction ingredient for *Sargassum macrocarpum* can be detected or not in the other cell system except PC12D cells, neuroblastoma Neuro-2a cells derived from mouse neuroblast were used for an examination.

### (1) Experimental method

The culturing of mouse neuroblastoma Neuro-2a cells was carried out by plating 5x10³ cells/100µl/well in a 96-well microplate under 95% air and 5% CO₂ at 37°C. As for the medium for cells, 10% HEPES buffer solution, ampicillin (2.5ng/ml), streptomycin (20 µg/ml) and penicillin (12 µg/ml) were added, followed by adjustment to pH 7.0 , and D-MEM medium (Gibco) containing 10% fetal bovine serum (FBS, Selumex) was used. After culturing for 6 days, as the measurement of the neurite elongation activity, the number of cells per one visual field and the number of cells having a neurite which was not less than 2 times larger than a cell body were calculated, and the neurite elongation activity for the methanol extraction ingredient for *Sargassum macrocarpum* under an optical microscope (x200 magnification) was evaluated according to the below calculation formula;
neurite elongation activity = number of cells having the neurite per 1 point/total number of cells per 1 point. Two wells per one sample were tested, and the neurite elongation activities were measured for 4 points per well , therefore, the visual fields of 8 points in a total were measured, to obtain the mean value. Further, as a negative control the neurite elongation activity for cells to which medium NGF 10ng/ml were added was measured, and the comparison was made.

### (2) Results

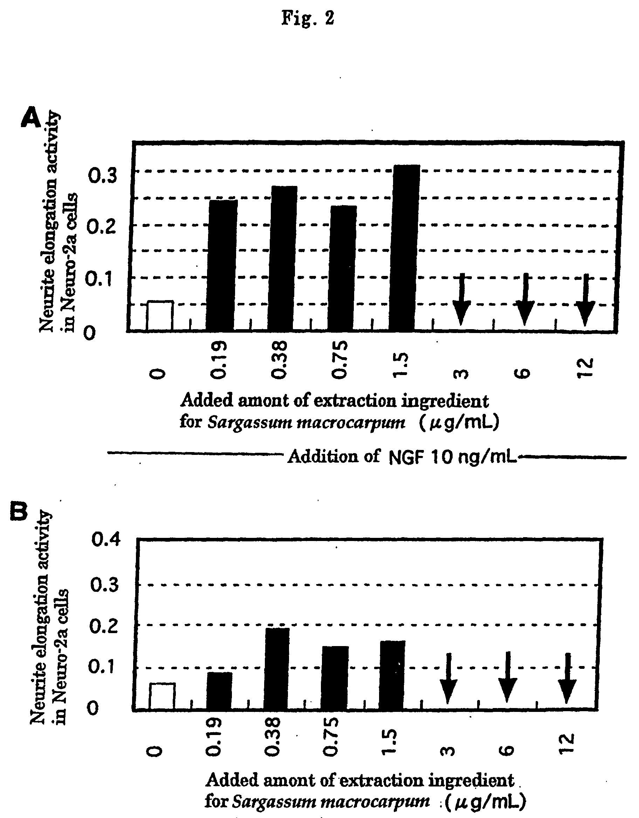

As shown in Fig. 2A, in the negative control group added with NGF 10ng/ml the neurite elongation activity was approximately 0.05, and on the contrary, the dose dependent neurite elongation activity potentiation by addition of the methanol extraction ingredient for *Sargassum macrocarpum* in the range of 0.19-1.5 µg/ml was shown. In the addition of the lowest dose 0.19 µg/ml, approximately 5 times higher activity was shown compared with the negative group, and in the addition of the highest dose 1.5 µg/ml, approximately 6 times higher activity was shown. very interestingly, in Neuro-2a cells even the methanol extraction ingredient for *Sargassum macrocarpum* only shows the potentiation in the absence of NGF in contrast to PC12D cells, whereby approximately 3 times higher activity was shown in the addition of 0.38 µg/ml compared with the control group (Fig. 2B). Based on the above, it became clear that the methanol extraction ingredient for *Sargassum macrocarpum* potentiated the neurite elongation activity of mouse neuroblastoma Neuro-2a cells, and it was considered that the methanol extraction ingredient for *Sargassum macrocarpum* showed the neurite elongation activity potentiation regardless of cell species.

### 7. Identification of methanol extraction ingredient for Sargassum macrocarpum

The methanol extraction ingredient for *Sargassum macrocarpum,* brown alga, was examined by NMR spectrum and was, confirmed to be 2,6,10-dodecatrienoic acid.
1H-NMR spectrum: (600MHz, CD3OD) δ=6.566 (1H, d, J3, 2-Me=1.5Hz, 3-H), 6.433 (1H, d, 5,1'=1.6Hz,5-H), 5.0808 (1H,t,J9',10'=7.3Hz,10'-H), 5.179 (1H, t, J1', 2'=7.3Hz, 2'-H), 5.130 (1H, t, J5', 6'=7.0Hz, 6'-H), 5.087 (1H, t, J13', 14'=7.0Hz, 14'-H), 3.123 (2H, d, J1', 2'=7.3Hz, 1'-H), 2.502 (2H, m, 9'-H), 2.210 (2H, m, 12'H), 2.170-2.130 (2H, m, 5'-H), 2.120-2.030 (6H, m, 4',8' and 13'-H), 2.027 (3H, d, J3, 2-Me=1.5Hz, 2-Me), 1.661 (3H, s, 16'-H), 1.649 (3H, s, 17'-H), 1.600 (3H, s, 18'-H), 1.575 (3H, s, 20'-H).
13C-NMR spectrum: (100MHz, CD3OD) δ=189.5 (1-C), 188.9 (4-C), 172.0 (19'-C), 150.0 (6-C), 147.6 (2-C), 141.9 (10'-C), 140.5 (3'-C), 135.9 (7'-C), 134.0 (3-C), 133.9 (11'-C), 133.1 (5-C), 132.9 (15'-C), 125.7 (6'-C), 124.8 (14'-C), 120.1 (2'-C), 40.7 (4'-C), 40.3 (8'-C), 36.1 (12'-C), 29.1 (9'-C), 29.0 (13'-C), 28.6 (1'-C), 27.3 (5'-C), 25.9 (16'-C), 17.8 (20'-C), 16.2, 16.1, 16.0 (18'-C).
HRMS (EI), Found: m/s 424.2613, Calcd for C₂₇H₃₆O₄ (M): 424.2613

The same data were obtained on *Jania adharens*.

### 8. Preparation examples

In the following, the dodecatrienoic acid derivatives, more particularly, the formulation examples of preparations prepared by using the dodecatrienoic acid derivatives shown by the general formula (I) are described. Further, this preparation formulation example is only one example, and it is to be understood that various preparation formulations can be made according to a known method.

### Preparation formulation 1

An aqueous injection is prepared by the following blending formulation.

| | |
|---|---|
| 2,6,10-dodecatrienoic acid (Na salt) | 1.0g |
| Benzyl alcohol | 2.0g |
| Nicotinamide | 3.0g |
| Propylene glycol | 40.0g |
| Distilled water | 100ml |

### Preparation formulation 2

An injection of lipid emulsion is prepared by the following blending formulation.

| | |
|---|---|
| 2,6,10-dodecatrienoic acid (Na salt) | 1.0g |
| Soybean oil (Japanese Phrmacopeia) | 21.0g |
| Purified soybean phospholipid | 2.5g |
| Glycerin | 5.0g |
| Distilled water | 175ml |

### Preparation formulation 3

A tablet is prepared by the following blending formulation (the total amount, 100 wt.%).

| | |
|---|---|
| 2,6,10-dodecatrienoic acid | 25.0 wt.% |
| Fumaric acid | 10.0 wt.% |
| Dibasic calcium phosphate | 40.0 wt.% |
| Lactose | 24.0 wt.% |
| Talc | 1.0 wt.% |

The tablet was subjected to an application of spray coating of a coating liquid consiting of ethyl cellulose, polyvinyl pyrrolidone K30, talc and ethyl alcohol according to a conventional method to prepare sustained release tablets.

### Preparation formulation 4

A suppository is prepared by the following blending formulation (the total amount, 100 wt.%).

| | |
|---|---|
| 2,6,10-dodecatrienoic acid | 5.0 wt.% |
| Propylene glycol | 6.0 wt.% |
| White beeswax | 10.0 wt.% |
| Sorbitan sesquioleate | 4.49 wt.% |
| Medium chain fatty acid triglyceride | 74.5 wt.% |
| Dibutyl hydroxy toluene | 0.01 wt.% |

### Preparation formulation 5

An ointment is prepared by the following blending formulation (the total amount, 100 wt.%).

| | |
|---|---|
| 2,6,10-dodecatrienoic acid | 5.0 wt.% |
| Propylene glycol | 6.5 wt.% |
| Isopropyl myristate | 5.5 wt.% |
| White petrolatum | 83.0 wt.% |

### Preparation formulation 6

A liniment is prepared by the following blending formulation (the total amount, 100 wt.%).

| | |
|---|---|
| 2,6,10-dodecatrienoic acid | 3.0 wt.% |
| Ethanol | 38.0 wt.% |
| 2-Hydroxy-4-methoxybenzophenone | 0.5 wt.% |
| Propylene glycol | 13.0 wt.% |
| Methyl cellulose | 0.8 wt.% |
| Ethyl sebacate | 3.0 wt.% |
| Purified water | appropriate |
| Sodium hydroxide | 0.07 wt.% |

### Preparation formulation 7

A gel is prepared by the following blending formulation (the total amount, 100 wt.%).

| | |
|---|---|
| 2,6,10-dodecatrienoic acid | 3.0 wt.% |
| Diisopropyl adipate | 3.0 wt.% |
| Ethanol | 38.5 wt.% |
| Carboxyvinyl polymer | 2.0 wt.% |
| Purified water | appropriate |
| Hydroxypropyl cellulose | 2.0 wt.% |
| Propylene glycol | 17.0 wt.% |
| Diisopropanolamine | 2.5 wt.% |

### Preparation formulation 8

A gelatinized cream is prepared by the following blending formulation (the total amount, 100 wt.%).

| | |
|---|---|
| 2,6,10-dodecatrienoic acid | 3.0 wt.% |
| Isopropyl myristate | 11.0 wt.% |
| Ethanol | 6.0 wt.% |
| Carboxyvinyl polymer | 1.5 wt.% |
| Purified water | appropriate |
| Polyoxyethylene (55) monostearate | 1.0 wt.% |
| Coconut fatty acid ethanolamide diethanolamide | 4.0 wt.% |

### Preparation formulation 9

A cream is prepared by the following blending formulation (the total amount, 100 wt.%).

| | |
|---|---|
| 2,6,10-dodecatrienoic acid | 5.0 wt.% |
| Cetyl alcohol | 12.0 wt.% |
| Stearyl alcohol | 2.5 wt.% |
| Glyceryl monostearate | 6.0 wt.% |
| 1,3-butylene glycol | 13.0 wt.% |
| Purified water | appropriate |

### Preparation formulation 10

A fomentation is prepared by the following blending formulation (the total amount, 100 wt.%).

| | |
|---|---|
| 2,6,10-dodecatrienoic acid | 3.0 wt.% |
| Gelatin | 6.0 wt.% |
| Aluminum silicate | 11.0 wt.% |
| Polyvinyl alcohol | 4.5 wt.% |
| Purified water | appropriate |
| Glycerin | 28.0 wt.% |
| Carboxymethyl cellulose | 3.0 wt.% |

### Preparation formulation 11

A plaster is prepared by the following blending formulation (the total amount, 100 wt.%).

| | |
|---|---|
| 2,6,10-dodecatrienoic acid | 3.0 wt.% |
| Styrene-isoprene-styrene block copolymer (Kalyflex TR1107, manufactured by Shell Kagaku K.K.) | 24.5 wt.% |
| Liquid paraffin | 43.5 wt.% |
| Hydrogenated rosin ester | 29.0 wt.% |

### Preparation formulation 12

A nasal drops are prepared by the following blending formulation (the total amount, 100 wt.%).

| | |
|---|---|
| The compound of the sample No.7 | 10 mg |
| Propylene glycol | 20 mg |

Distilled water: appropriate (The total amount was made 100 ml.)

### [Industrial Applicability]

The dodecatrienoic acid derivatives potentiate the neurite elongation action of NGF and are useful as a nerve growth factor activity potentiator . Therefore, said derivatives are useful as an ingredient for a medicine to treat Alzheimer type dementia.

## Claims

1. A nerve growth factor activity potentiator which contains as an active ingredient dodecatrienoic acid derivatives represented by the general formula (I): 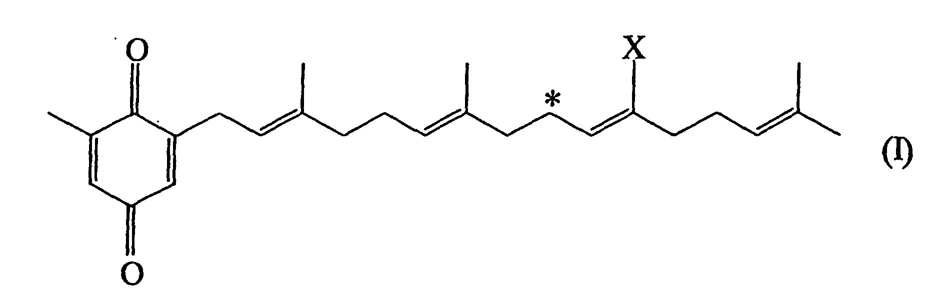 wherein X represents hydrogen, CHO, COOH, COOR (wherein R represents an ester residue or an addition salt comprising an alkali metal salt or an organic acid salt group), provided that in case X is COOH, it is bonded to the carbon atom at the position marked with * so that it forms a lactam O=C-O-.

2. A nerve growth factor activity potentiator comprising an extraction ingredient extracted from *Sargassum macrocarpum* or *Jania adharens.*

3. A nerve growth factor activity potentiator wherein the extraction ingredient according to claim 2 contains 2,6,10-dodecatrienoic acid or salts thereof.

4. A pharmaceutical composition for a dementia disease containing the agent potentiating nerve growth factor activity according to claims 1-3.
